# EUROPEAN PATENT SPECIFICATION

(11) **EP 4 507 641 B1**
(45) Date of publication and mention of the grant of the patent: **21.01.2026**
(21) Application number: 23723642.7
(22) Date of filing: 06.04.2023
(51) Int. Cl.: A61F 9/008, A61B 3/10, A61B 3/12

(54) **SLO-BASED LASER GUIDANCE FOR TREATING VITREOUS FLOATERS**
SLO-BASIERTE LASERFÜHRUNG ZUR BEHANDLUNG VON GLASKÖRPERSCHWIMMERN
GUIDAGE LASER À BASE DE SLO POUR LE TRAITEMENT DE CORPS FLOTTANTS DU VITRÉ

(30) Priority: 12.04.2022 US 202263330141 P
(43) Date of publication of application: 19.02.2025
(73) Proprietor: Alcon Inc., 1701 Fribourg (CH)
(72) Inventor: BOR, Zsolt, Lake Forest, California 92630 (US); MALEK TABRIZI, Alireza, Freemont, California 94539 (US)
(74) Representative: Murgitroyd & Company
(86) International application number: PCT/IB2023/053555
(87) International publication number: WO 2023/199187

(56) References cited:
- WO-A1-2021/069168
- US-A1- 2016 074 221
- US-A1- 2018 317 767
- US-A1- 2020 038 241
- MILSTON REBECCA ET AL: "Vitreous floaters: Etiology, diagnostics, and management", SURVEY OF OPHTHALMOLOGY, vol. 61, no. 2, 8 December 2015 (2015-12-08), pages 211 - 227, XP029439479, ISSN: 0039-6257, DOI: 10.1016/J.SURVOPHTHAL.2015.11.008

## Description

### TECHNICAL FIELD

The present disclosure relates generally to ophthalmic surgical laser systems, and more particularly to confocal SLO-based laser guidance for treating vitreous floaters.

### BACKGROUND

Eye floaters are clumps of collagen proteins that form in the vitreous. These clumps can disturb vision with moving shadows. In laser vitreolysis, a laser beam is directed into the vitreous to fragment the floaters to improve vision.

Reference is made to the documents US 2016/074221 Al and MILSTON R et at: "Vitreous floaters: Etiology, diagnostics, and management", SURVEY OF OPHTHALMOLOGY, vol. 61, no. 2, 8 December 2015 (2015-12-08), pages 211-227, which have been cited as exemplary of the background state of the art.

### BRIEF SUMMARY

The scope of the invention is in accordance with the appended claims.

In certain embodiments, an ophthalmic surgical laser system for treating a floater in a vitreous of an eye includes a scanning laser ophthalmoscope (SLO) subsystem, a treatment laser subsystem, a scanner, optical elements (including focusing lenses), and a computer. The SLO subsystem includes an SLO laser source and a pinhole filter. The SLO laser source provides an SLO laser beam with an SLO focal point, and the pinhole filter is conjugated to the SLO focal point. The treatment laser subsystem provides a treatment laser beam with a treatment focal point that spatially coincides with the SLO focal point. The scanner scans the SLO laser beam across a scan region and directs the treatment laser beam to the xy-location of the scan region. The optical elements aim the SLO laser beam and the treatment laser beam at substantially the same point of the scan region, focus the SLO focal point to form the scan region at a z-scan location, move the scan region to the z-scan location of the floater, and focus the treatment focal point at the z-scan location of the floater. The computer receives an image of the floater from the SLO subsystem, determines an xy-location of the floater, and instructs the treatment laser subsystem to direct the treatment laser beam towards the xy-location and the z-scan location of the floater.

Embodiments may include none, one, some, or all of the following features:
The optical elements move the scan region in the z-direction by changing the relative distance between the eye and at least one optical element.
* The optical elements move the scan region in the z-direction by changing the relative distance between the focusing lenses.
* The ophthalmic surgical laser system includes a tunable lens that moves the SLO focal point and the treatment focal point. The optical elements move the scan region in the z-direction by adjusting the tunable lens to move the SLO focal point and the treatment focal point.
* The computer determines the xy-location of the floater by receiving user input indicating the xy-location of the floater.
* The computer determines the xy-location of the floater by analyzing the image of the floater from the SLO subsystem and determining the xy-location of the floater from the image.
* The optical elements move the scan region to the retina of the eye, and the SLO subsystem generates an image of a shadow of the floater on the retina.
* The treatment laser subsystem directs the treatment laser beam towards the xy-location and the z-scan location of the floater by directing a laser pulse at the floater.
* The treatment laser subsystem directs the treatment laser beam towards the xy-location and the z-scan location of the floater by directing laser pulses towards the floater to form a three-dimensional volume covering 80% or more of the volume of the floater.
* The computer analyzes an image of a shadow on the retina of the eye from the SLO subsystem and determines whether the shadow is cast by a significant floater.
* The computer analyzes an image of the floater from the SLO subsystem and determines whether the floater is in focus.
* The computer calculates a floater-to-retina distance according to changes in the system used to adjust the scan region between the retina and the floater. The computer may calculate the floater-to-retina distance according to the distance system components move to adjust the scan region between the retina and the floater, or according to the difference in diopters used to adjust the scan region between the retina and the floater.
* The computer calculates a retinal radiation exposure of the treatment laser beam focused at the z-scan location of the floater, and performs an alarm response if the retinal radiation exposure exceeds a predetermined limit. The computer may calculate the retinal radiation exposure EXP according to EXP= E/[(L*α)²π/4], where E represents a laser pulse energy, α represents a full convergence angle of the treatment laser beam focused at the z-scan location of the floater, and L represents a floater-to-retina distance calculated according to the z-scan location of the floater.
* The computer tracks movement of the floater using tracking software.
* The SLO subsystem generates images of the eye at different z-scan locations, and the computer generates a three-dimensional image from the images.
* The system further comprises a display. The display may be a computer monitor or virtual reality glasses.
* The treatment laser beam has a wavelength in the range of 350 nanometers (nm) to 2000 nm and laser pulses with pulse durations in the range of 20 femtoseconds (fs) to 1000 nanoseconds (ns).
* The system further comprises an ultrasonic sensor that monitors the position of the eye and/or head of the patient relative to an objective lens.

As illustrative example, not part of the claimed invention, a method for treating a floater in a vitreous of an eye is described, that includes : providing, by a scanning laser ophthalmoscope (SLO) subsystem, an SLO laser beam with an SLO focal point; providing, by a treatment laser subsystem, a treatment laser beam with a treatment focal point, the treatment focal point spatially coinciding with the SLO focal point; scanning, by a scanner, the SLO laser beam across a scan region; aiming, by optical elements, the SLO laser beam and the treatment laser beam at substantially the same point of the scan region; focusing, by the optical elements, the SLO focal point to form the scan region at a z-scan location; moving, by the optical elements, the scan region to the z-scan location of the floater; receiving, by a computer, an image of the floater from the SLO subsystem; determining, by the computer, an xy-location of the floater; instructing, by the computer, the treatment laser subsystem to direct the treatment laser beam towards the xy-location and the z-scan location of the floater; directing, by the scanner, the treatment laser beam to an xy-location of the scan region; and focusing, by the optical elements, the treatment focal point at the z-scan location of the floater.

These methods may include none, one, some, or all of the following features:

* The scan region is moved in the z-direction by changing the relative distance between the eye and at least one optical element.

* The scan region is moved in the z-direction by changing the relative distance between the focusing lenses.

* The scan region is moved in the z-direction by adjusting a tunable lens to move the SLO focal point and the treatment focal point.

### BRIEF DESCRIPTION OF THE DRAWINGS

FIGURES 1A and 1B illustrate an example of an ophthalmic surgical laser system that can move a scan region to different z-locations;
FIGURES 2A through 2C illustrate examples of images that may be generated at different z-locations;
FIGURE 3 illustrates an example of an ophthalmic surgical laser system that may be used to treat a floater in an eye, according to certain embodiments;
FIGURE 4 illustrates an example of how an ophthalmic surgical laser system can move a scan region, according to certain embodiments;
FIGURE 5 illustrates another example of how an ophthalmic surgical laser system can move a scan region, according to certain embodiments;
FIGURE 6 illustrates yet another example of how an ophthalmic surgical laser system can move a scan region, according to certain embodiments;
FIGURES 7A to 7C illustrate examples of images of a floater that were generated at different z-scan locations; and
FIGURE 8 illustrates an example of a method for treating a floater that may be used by the system of FIGURE 1.

### DESCRIPTION OF EXAMPLE EMBODIMENTS

Referring now to the description and drawings, example embodiments of the disclosed apparatuses and systems are shown in detail. The description and drawings are not intended to be exhaustive or otherwise limit the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments.

In certain laser vitreolysis systems, an OCT device is used to provide the z-location of a floater to guide a treatment laser beam towards the floater. OCT devices, however, are complex and expensive. Accordingly, embodiments described herein do not use an OCT device to provide the z-location of the floater.

Examples of a system described herein provide a scanning laser ophthalmoscope (SLO) laser beam to generate an SLO image of a floater or a retinal floater shadow and a treatment laser beam to disintegrate the floater. The system collimates and aligns both the SLO and treatment laser beams, so the focal points of the beams are focused and aimed at the substantially the same location within the eye. In the embodiments, the focal point of the SLO laser beam can be moved in the z-direction to image the floater itself. When the floater image is in focus, the treatment beam is also focused on the floater since the SLO and treatment focal points are aimed at the same location. Thus, an SLO device can be used to direct a treatment beam towards the z-location of a floater, without assistance from an OCT device.

### 1. Example Surgical System

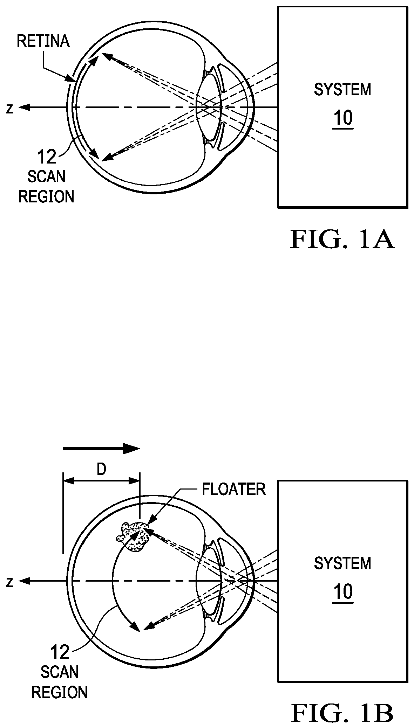

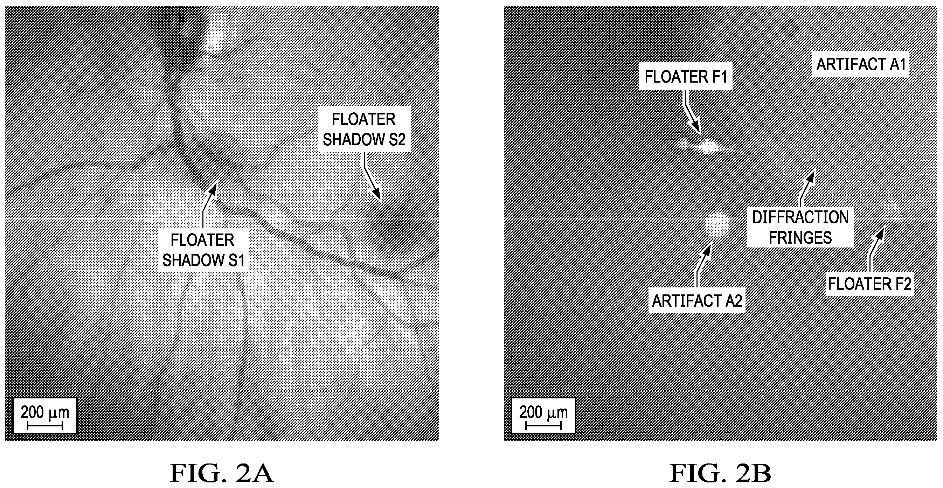

FIGURES 1A and 1B illustrate an example of an ophthalmic surgical laser system 10 that can move a scan region 12 to different z-locations, e.g., from the retina to a floater, and FIGURES 2A and 2B illustrate examples of SLO images that may be generated at the different z-locations. Scan region 12 is the focal surface indicating where the focal points of SLO and treatment laser beams are aimed. In the example, the z-location of scan region 12 may be described as the "z-scan location". For example, the z-scan location of scan region 12 in FIGURE 1A is at the retina, and the z-scan location of scan region 12 in FIGURE 1A is at the floater.

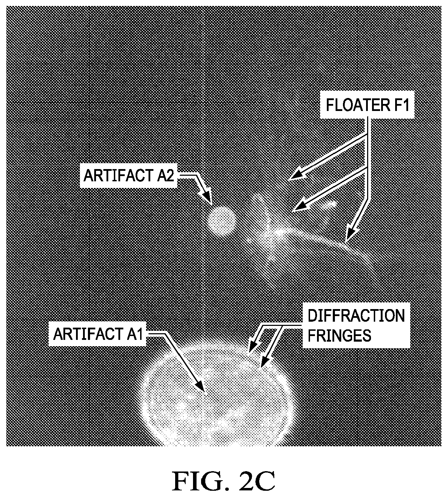

In FIGURES 1A and 2A, scan region 12 is located at the retina. FIGURE 2A shows shadows S1 and S2 that floaters cast on the retina. In FIGURES 1B, 2B, and 2C, scan region 12 is located at a floater F1.

In FIGURE 2B, since scan region 12 is located at floater F1, floater F1 appears as a bright, sharp, two-dimensional enface object moving within the image frame. Floater F2 is near, but not at, the focus of the SLO beam, so appears darker and blurrier than floater F1. In general, the shape of a floater should resemble the shape of the corresponding floater shadow. Artifact A1 is a first Purkinje image caused by a reflection from the anterior surface of the cornea. Artifact A1 includes diffraction fringes around the edge of the first Purkinje image. Artifact A2 is caused by reflections from optical components (such as L1, L2, L3, L4, and/or L5).

In FIGURE 2C, floater F1 is a large, diffuse floater at the focal point of the SLO beam. Since scan region 12 is located at floater F1, floater F1 appears as a bright, sharp, two-dimensional enface object moving within the image frame. As described above, artifact A1 is a first Purkinje image, and artifact A2 is caused by reflections from optical components.

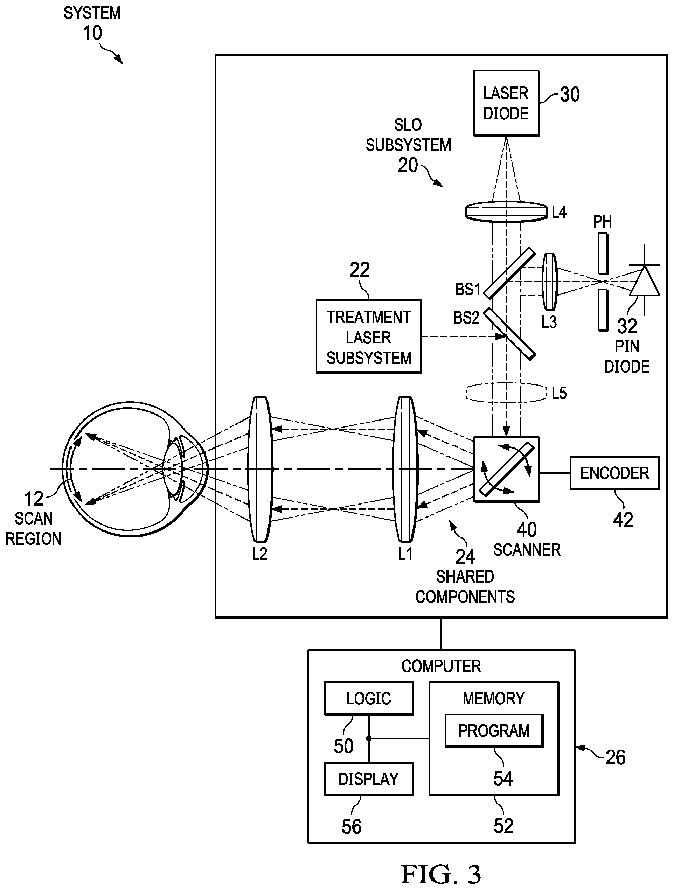

FIGURE 3 illustrates an example of an ophthalmic surgical laser system 10 that may be used to image and treat a floater in an eye, according to certain embodiments. As an overview, system 10 includes a scanning laser ophthalmoscope (SLO) subsystem 20, a treatment laser subsystem 22, one or more shared components 24, and a computer 26, coupled as shown. SLO subsystem include a laser source (e.g., laser diode 30), optical elements (such as lenses L3, L4, and/or L5, and beamsplitters BS1 and BS2), a pinhole filter PH, and a PIN diode 32, coupled as shown. Shared components 24 include a scanner (e.g., an xy- or 2D scanner) 40, an xy-encoder 42, and optical elements (such focusing lenses L1 and L2, where L2 may be an objective lens), coupled as shown. Computer 26 includes logic 50, a memory 52 (which stores a computer program 54), and a display 56, coupled as shown. System 10 may include a sensor (e.g., an ultrasonic sensor) that monitors the position of the patient's eye and/or head relative to a system component (e.g., objective lens L2). In general, an optical element can act on (e.g., transmit, reflect, refract, diffract, collimate, condition, shape, focus, modulate, and/or otherwise act on) a laser beam.

According to an overview of an example of imaging, e.g., the retina or a floater, an SLO laser source (e.g., laser diode 30) of SLO subsystem 20 provides an SLO laser beam with an SLO focal point. Lens L4 collimates the SLO laser beam. The SLO laser beam passes through beamsplitters BS1 and BS2, is deflected by scanner 40, passes through lenses L1 and L2 (and L5 in certain embodiments), and is focused onto, e.g., the retina or a floater. That is, beamsplitter BS1 passes the SLO laser beam through towards beamsplitter BS2, and beamsplitter BS2 passes the beam through towards scanner 40. (In certain embodiments, tunable lens L5 passes the SLO laser beam through.) Scanner 40 scans the SLO laser beam across a scan region within the eye. Focusing lenses L1 and L2 image scanner 40 into the pupil to focus the SLO and treatment laser beams to a common focal point to form the scan region at the z-scan location of, e.g., the retina or a floater, which reflects back the light. In this way, a large angular range of the retina can be scanned with the SLO beam.

Continuing with the example overview, the reflected light travels back through lenses L1, L2, scanner 40, (lens L5 in certain embodiments), and beamsplitter BS2. Beamsplitter BS1 directs the reflected beam towards lens L3, which focuses the beam onto confocal pinhole filter PH. Pinhole filter PH directs the beam to PIN diode 32. The position of pinhole filter PH is optically conjugated to the focal point of the SLO laser beam, so only light reflected back from the focal point of the SLO beam is detected by PIN diode 32 and all other back reflections are suppressed by pinhole filter PH. PIN diode 32 outputs a signal proportional to the intensity of the detected light, which can be used to generate an image of the floater.

Continuing with the example overview, computer 26 gathers the signal from PIN diode 32 and the angular deflections of the beams from xy-encoder 42 and generates an image of the reflected light as a function of the xy-readings from xy-encoder 42. If system 10 is imaging and treating a floater in the vitreous, optical elements are positioned to aim the SLO and treatment laser beams the z-location of the floater. The floater image is sharper and brighter while the background is darker because pinhole filter PH allows backscattered light from the floater through to PIN diode 32 and suppresses backscattered light from the retina. Computer 26 determines the xy-location of the floater, and instructs treatment laser subsystem 22 to generate the treatment laser beam and scanner 40 to aim the beam at the xy-location of the floater. Beamsplitter BS1 directs the treatment laser beam to optical elements that direct the beam to the z-scan location of the floater to destroy the floater.

Turning to the parts of the system, SLO subsystem 20 utilizes confocal laser scanning to generate images of the interior of the eye. SLO subsystem 20 moves scan region 12 of SLO focal point in the z-direction to gather light at different z-scan locations, e.g., from the retina and through the vitreous. Pinhole filter PH is optically conjugated to the SLO focal point such that only the light from the SLO focal point is detected by PIN diode 32 and the other light (e.g., reflections from the cornea or lens) is suppressed. SLO subsystem 20 plots the intensity of the back reflected light as a function of the xy-position of the focal point to generate an SLO image.

Treatment laser subsystem 22 generates a laser beam with any suitable wavelength, e.g., in a range from 350 nm to 2000 nm. Treatment laser subsystem 22 delivers laser pulses at any suitable repetition rate (e.g., a single pulse to 200 megahertz (MHz)). A laser pulse has any suitable pulse duration (e.g., 20 femtoseconds (fs) to 1000 nanoseconds (ns)), any suitable pulse energy (e.g., 1 nanojoule (nJ) to 10 millijoule (mJ) or an energy that exceeds the optical breakdown threshold of the vitreous), and a focal point of any suitable size (e.g., 1 to 30 microns). The treatment laser beam is collimated to spatially coincide with the SLO beam such that the focal points of the SLO beam and the treatment laser beam precisely spatially coincide, i.e., the beams are focused at the same spot.

In certain embodiments, treatment laser subsystem 22 directs a laser pulse at the floater to destroy the floater. In other embodiments, treatment laser subsystem 22 directs a plurality of laser pulses that form a three-dimensional (3D) volume. The geometric center of the 3D volume may be substantially aligned with the geometric center of the floater, and the 3D volume may cover all or almost all, such as 80% or more of the volume of the floater. In the embodiments, scanner 40 scans the pulses in the xy-directions, and, e.g., tunable lens L5 scans the focus in the z-direction.

Shared components 24 direct beams from SLO subsystem 20 and treatment laser subsystem 22, towards the eye. Because SLO and laser beams share components 24, the beams are affected by the same geometrical distortions (e.g., fan distortion of scanners, barrel or pillow distortions of the scanner lens, refractive distortions from the inner eye surfaces, and other distortions). The distortions affect the beams in the same way, so the beams propagate along the same path. This allows for aiming the laser beam precisely at the floater. Shared components 24 may also provide spectral and/or polarization coupling and/or decoupling of the SLO and laser beams to allow the beams to share the same path.

Turning to the details of shared components 24, scanner 40 may be any suitable xy-scanner that changes the angle of incidence of a beam into the pupil to angularly direct the focal point of the beam in the x- and y-directions. Scanner 40 may include, e.g., a pair of galvanometrically-rotated scanner mirrors that can be tilted about mutually perpendicular axes; an acousto-optical crystal that can acousto-optically steer the beam; and/or a fast scanner (e.g., a galvanometric, resonant, or acousto optical scanner) that can create, e.g., a two-dimensional matrix of laser spots of the treatment laser beam.

Xy-encoder 42 detects the angular position of xy-scanner 40 and reports the position as the xy-location measured in encoder units, e.g., angular units. For example, xy-encoder 42 detects the angular orientations of the galvanometer mirrors of xy-scanner 40 and records the orientations as encoder units. Xy-encoder 42 may report the position in encoder units to SLO subsystem 20, treatment laser subsystem 22, and/or computer 26. Since both SLO subsystem 20 and treatment laser subsystem 22 share the same xy-scanner 40, computer 26 can use the encoder units to instruct subsystems 20, 22 where to aim their beams, making it unnecessary to perform the computer-intensive conversion from encoder units to a length unit such as millimeters. Xy-encoder 42 reports the positions at any suitable rate, e.g., once every 5 to 50 milliseconds (ms), such as every 10 to 30 or approximately every 20 ms.

Lenses L1 and L2 image scanner 40 into the pupil to allow the SLO and treatment laser beams to scan a wide angular range. Lenses L1 and L2 include any suitable number of lenses arranged in any suitable manner to image scanner 40 into the pupil while allowing system 10 to change the convergence angle (i.e., the diopter number) of the beam entering the pupil. The diopter can be increased to shift the focus of the SLO beam into the vitreous.

Computer 26 controls components of system 10 (e.g., SLO subsystem 20, laser device 24, and/or shared components 24) in accordance with a computer program 54. Computer 26 may be separated from components or may be distributed among system 10 in any suitable manner, e.g., within SLO subsystem 20, laser device 24, and/or shared components 24. In certain embodiments, portions of computer 26 that control SLO subsystem 20, laser device 24, and/or shared components 24 may be part of SLO subsystem 20, laser device 24, and/or shared components 24, respectively.

Computer 26 controls the components of system 10 in accordance with a computer program 54. Examples of computer programs 54 include object imaging, object tracking, image processing, image analysis, floater evaluation, and retinal exposure calculation programs. For example, computer 26 uses a computer program 54 to instruct SLO subsystem 20, laser device 24, and/or shared components 24 to: move the scan region to a floater such that the SLO and treatment focal points are aimed at the floater; generate an image of the floater; determine the xy-location of the floater; and/or direct the treatment laser beam towards the floater.

In certain embodiments, computer 26 uses an image analysis program 54 to analyze the digital information of images. For example, computer 26 analyzes images using, e.g., edge detection or pixel analysis, to look for shapes that could be floaters and differentiate between floater and non-floater images. Computer 26 may identify a darker shape on the retina moving relative to the vasculature as a floater shadow and a lighter shape in the vitreous as a floater. An opacity that moves with the vasculature may be recognized by computer 26 as an anatomical opacity of the retina, not a floater.

In addition, computer 26 may recognize certain images as artifacts, as shown in FIGURES 2B and 2C. A large, bright patch with diffraction fringes around the edge that moves in the opposite direction of the SLO image within the display frame (e.g., downwards when the image moves upwards in the display frame or to the left when the image moves to the right) may be a first Purkinje image, as shown as artifact A1 of FIGURES 2B and 2C. A bright spot that does not move relative to the SLO display frame may be an internal reflection from optical components (such as L1, L2, L3, L4, and/or L5), as shown as artifact A2 of FIGURES 2B and 2C.

As another example of using image analysis program 54, computer 26 analyzes the image of a floater shadow to determine if the shadow is cast by a significant floater, e.g., a clinically significant floater that should be treated. The shape and size of the floater shadow indicates the size and shape of the floater, and the tone or luminance of the floater shadow indicates the density of the floater. As another example, computer 26 analyzes the image of a floater to determine if the SLO focal point is focused on the floater. If the edges of floater are sharp, i.e., the floater is in focus, the focal point at or near the floater. If the floater image is the brightest when focusing the SLO beam in z direction and the sharpest, then the floater is at the focus of the SLO beam.

In certain embodiments, computer 26 generates images to be presented on display 56. For example, computer 26 presents multiple successive images as a video. As another example, computer 26 combines multiple SLO images taken a different z-scan locations into a three-dimensional (3D) image. The 3D image may be used to determine the spatial size and location of an array of laser treatment pulses used to treat a floater.

In certain embodiments, computer 26 determines the xy-location of the floater. For example, computer 26 may receive user input indicating the xy-location of the floater. The user may input the xy-location by selecting the xy-location on a displayed image that corresponds to the location of the floater, e.g., by touching the xy-location on a screen or by selecting the xy-location with a cursor. In other embodiments, computer 26 may perform image analysis on an SLO image to determine the xy-location of the floater. Computer 26 may identify the floater in the image and then determine the xy-location from the location in the image. After computer 26 determines the xy-location of the floater, computer 26 may instruct scanner 40 to direct a treatment beam to the floater location in encoder units.

In certain embodiments, computer 26 calculates the radiation exposure on the retina from a laser pulse directed towards a location, e.g., the location of a floater, and performs an alarm response if the retinal exposure exceeds a predetermined limit. For example, the retinal exposure EXP can be calculated as EXP= [E/(L*α)²π/4] where E is the laser pulse energy, α is the full convergence angle of the laser beam in the vitreous focused on the location of the floater, and L is the distance to the retina, e.g., the floater-to-retina distance, which can be calculated as described below. The radiant exposure should be less than a maximum radiant exposure, which may be determined in accordance with accepted standards. For example, the maximum radiant exposure may be set in accordance with ANSI Z80.36-2016 Maximum Permissible Exposure (ANSI MPE). If the radiant exposure exceeds the maximum radiant exposure, e.g., the ANSI MPE, computer 26 may perform an alarm response, such as modify any suitable factor (e.g., lower the pulse energy), provide a notification to the user, and/or prevent firing of the laser beam. Computer 26 may also calculate an exposure ratio R = EXP/[ANSI MPE] and provide ratio R to the user (e.g., surgeon) to allow the user to determine whether to treat the floater.

Display 56 may be any suitable hardware that can display an image. Examples of display 56 include a computer monitor and virtual reality glasses. A user (e.g., a surgeon or other medical personnel) at the surgery site or remote from the site may view images generated by the computer.

Involuntary and voluntary eye movements (e.g., saccadic and microsaccadic movements, drift, and tremor) can make laser treatment difficult. To reduce movement, the eye may be stabilized. For example, the treated eye and/or other eye may be stabilized with a fixation light. If the xy-position of the fixation light can be moved up to 25 degrees in any angular direction, floaters located far from the visual axis can be treated. As another example, a patient interface or handheld surgical contact lens may be used to mechanically stabilize the eye. The interface or lens may have a spherical contact surface that reduces refractive error of the eye, such as corneal astigmia and asphericity of the eye. As another example, movement of the floater may be tracked by tracking software, and the focus of the treatment laser may continuously be aimed at the moving floater.

### 2. Examples of Moving the Focal Point

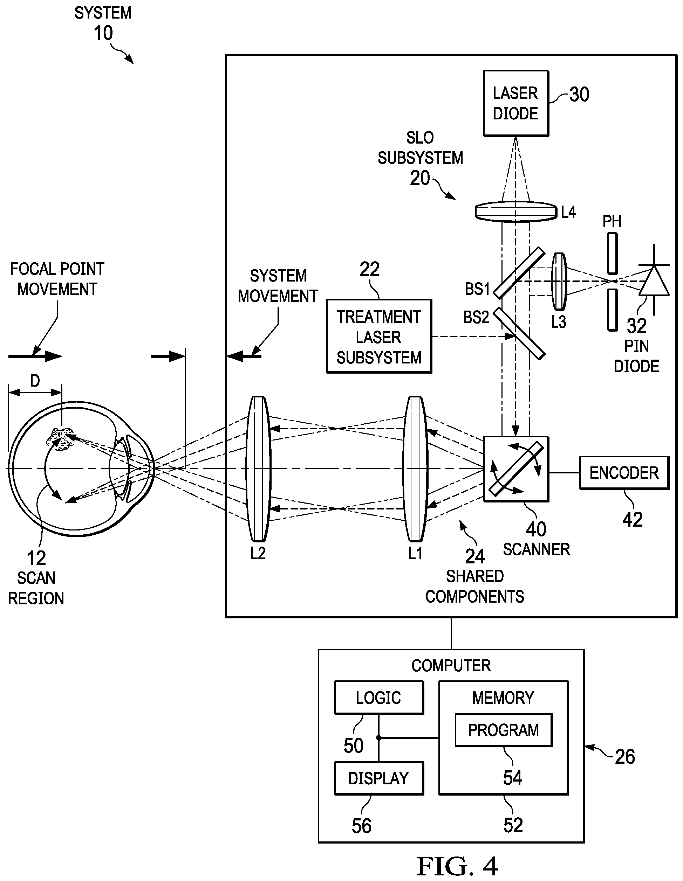

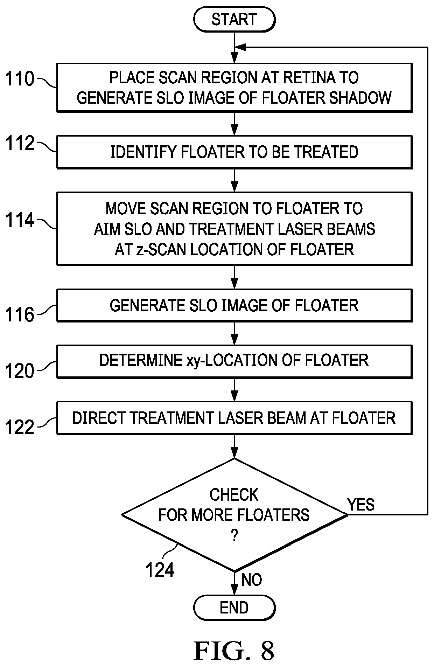

FIGURES 4 through 6 illustrate embodiments of ophthalmic surgical laser system 10 that can move the image and treatment focal points in the z-direction, and FIGURES 8A through 8C illustrate examples of images taken at different z-scan locations. In certain embodiments, components of system 10 are adjusted (e.g., moved, reshaped, or otherwise modified) to move scan region 12. The components may be adjusted in any suitable manner. For example, a mechanical structure can apply force to move a component in response to a user action; a motorized actuator can move a component; and/or an electrical current can reshape or otherwise modify a tunable lens. The movement may be initiated in response to, e.g., a user moving a controller (such as a knob) and/or a command from computer 26.

From the changes system 10 makes to adjust scan region 12 from the retina to a floater, computer 26 can calculate the distance between the retina and floater ("floater-to-retina distance"). For example, the floater-to-retina distance may be calculated from the distance components of system 10 move to adjust scan region 12 or the difference in diopters needed to adjust scan region 12, as described in more detail below. Computer 26 may use a mathematical function or a lookup table that describes the relationship between the floater-to-retina distance and the system changes (e.g., between the floater-to-retina distance and the distance components move to adjust scan region 12 or between the floater-to-retina distance and the difference in diopters needed to adjust scan region 12) to calculate the distance. The function or lookup table may be generated in any suitable manner. For example, during calibration or manufacture of system 10, the z-scan location can be recorded for different component locations or diopters, and the function or table can be determined from the results. As another example, the function or table may be determined from an eye model, such as a Zemax eye model. In certain embodiments, the floater-to-retina distance can be used to calculate the potential retinal radiation exposure from a laser beam directed towards the floater, as described above.

FIGURE 4 illustrates an example of how ophthalmic surgical laser system 10 can move scan region 12, according to certain embodiments. In the embodiments, computer 26 or the user moves scan region 12 by moving, relative to the eye, the optical elements that produce scan region 12, e.g., SLO subsystem 20, treatment laser subsystem 22, and/or shared components 24 (such as scanner 40 and focusing lenses L1, L2). The components may be mechanically coupled such that they move together in the z-direction relative to the base of system 10, e.g., a table base. In the embodiments, the eye is stabilized relative to the base, such that the movement (i.e., the change in z-location) of the components relative to the base represents movement M relative to the eye. Linear encoder 42 may record the relative movement M and report the relative movement M to computer 26 in encoder units.

In certain embodiments, computer 26 calculates the floater-to-retina distance F from the relative movement M. For example, each unit of relative movement M changes the z-scan location of scan region 12 by n units, where n is the refractive index of the vitreous, 1.34. Thus, floater-to-retina distance F is approximately F = 1.34 x M. As another example, during calibration of system 10, the z-scan location is recorded for different locations of the moving components, and a function or table is calculated from the results. Computer 26 uses the function or table to calculate the floater-to-retina distance F.

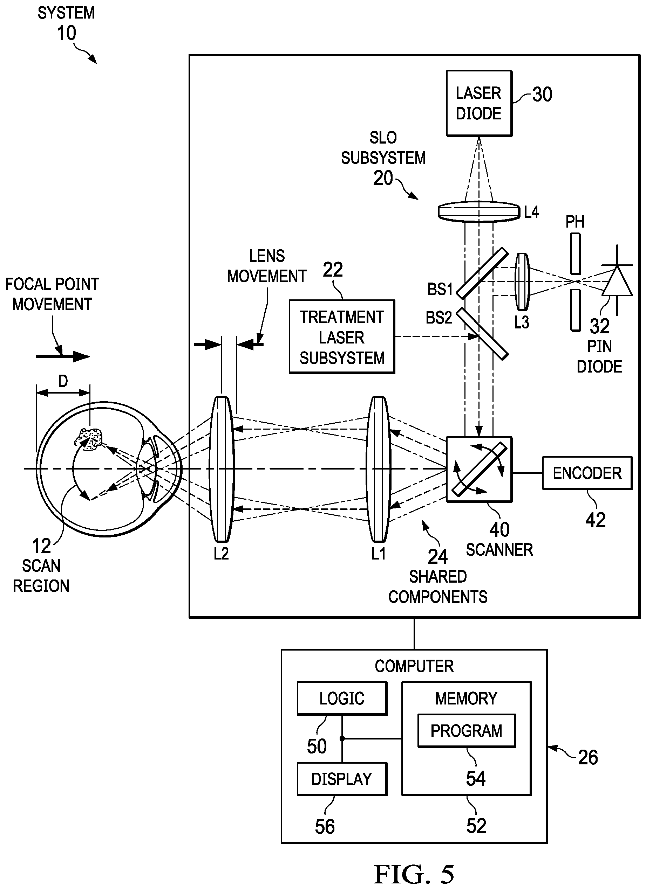

FIGURE 5 illustrates another example of how ophthalmic surgical laser system 10 can move scan region 12, according to certain embodiments. In certain embodiments, computer 26 moves the scan region by changing the relative distance between focusing lenses L1 and L2 to move the SLO and treatment laser focal points.

In certain embodiments, system 10 describes this change as a change in diopter D. In the embodiments, computer 26 calculates the floater-to-retina distance F from the change in diopter ΔD. For example, for the average human eye, each +1-diopter change ΔD in the convergence of the laser beam corresponds to a F = 0.36 mm shift of scan region 12 away from the retina. Thus, floater-to-retina distance F = 0.36 x ΔD mm. The 0.36 multiplier works in the vicinity of the retina, and lookup table or mathematical function may be generated, as described above, for larger distances.

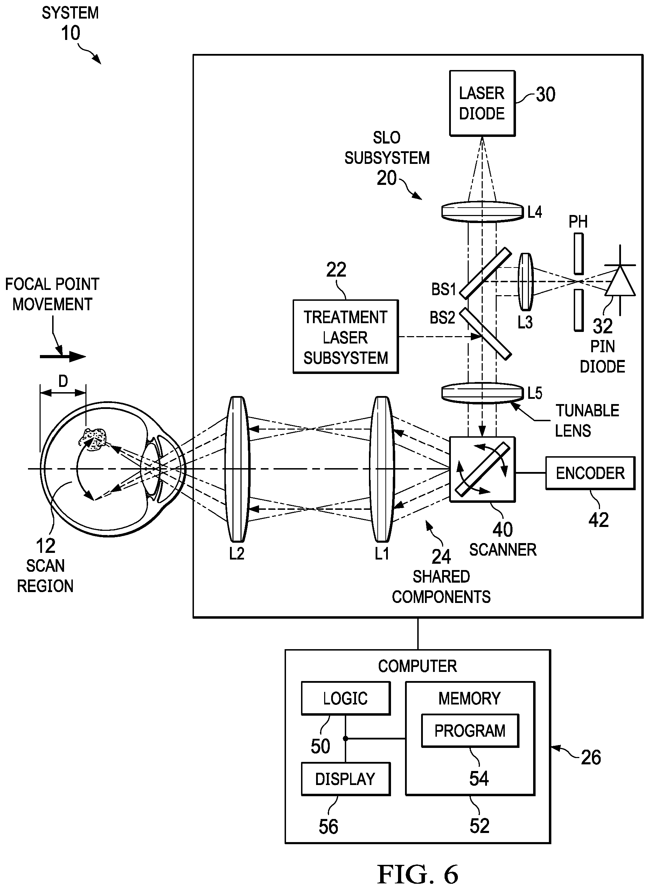

FIGURE 6 illustrates yet another example of how ophthalmic surgical laser system 10 can move scan region 12, according to certain embodiments. In certain embodiments, system 10 includes a tunable lens L5 that moves the SLO and treatment laser focal points in the z-direction. In the embodiments, computer 26 moves the scan region by adjusting the tunable lens L5 to convert the collimated laser beam to a convergent laser beam that forms the focal points. Tunable lens L5 may be any suitable lens or arrangement of lenses. For example, tunable lens L5 may be an electrically tunable lens, which may yield fast scanning of the focal points. As another example, tunable lens L5 may be a compound lens comprising two or more lenses. Adjusting the distance between the lenses changes the equivalent refractive power. In certain embodiments, system 10 describes this adjustment as a change in diopter D, and computer 26 calculates the floater-to-retina distance F from the change in diopter ΔD, as described above.

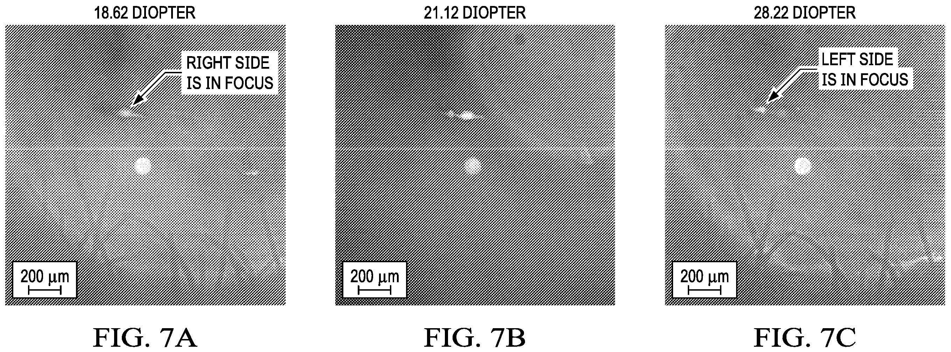

FIGURES 7A to 7C illustrate examples of images of the same floater that may be generated at different z-scan locations. In the images, different parts of the floater are in focus at different z-scan locations. FIGURE 7A shows the scan region at 18.6 D, or 6.7 mm from the retina, where the right side of the floater is in focus. FIGURE 7B shows the scan region at 21.2 D, or 7.6 mm, where the center is in focus. FIGURE 7C shows the scan region at 28.2 D, or 10.1 mm, where the right side is in focus.

### 3. Example Method

FIGURE 8 illustrates an example of a method for treating a floater that may be used by system 10 of FIGURE 1. Certain steps of the method may be performed by the computer of the system instructing system components to perform the steps. During the method, the computer may calculate a retinal radiation exposure of the treatment laser beam on the retina. If the retinal radiation exposure exceeds a predetermined limit, the computer may perform an alarm response (e.g., notify the user and/or stop the treatment).

The method starts at step 110, the scan region is placed at the retina to generate an SLO image of a floater shadow on the retina. The computer or user may adjust (e.g., move) optical elements that produce the scan region to place the scan region to the retina. The scanner of the system may perform an angular scan around the visual axis larger than, e.g., 45 degrees, to detect floater shadows. The floater to be treated is identified at step 112. In certain embodiments, the computer may analyze an image of the floater shadow to determine if the floater may be graded as clinically significant according to, e.g., the size, density, and/or xy-location of the floater shadow. In other embodiments, the user (e.g., a surgeon) may determine if the floater is significant.

The scan region is moved to the floater to aim the SLO and treatment laser beams towards the z-scan location of the floater at step 114. The computer or user may adjust optical elements that produce the scan region in order to move the scan region. The optical element may be adjusted by, e.g., moving one or more of the optical elements, changing the relative distance between focusing lenses of the focal points, and/or adjusting a tunable lens that moves the focal points. When the scan region is at the floater, the image of the floater is bright and sharp because the focal points of the SLO and the treatment laser beams are at the floater. The brightness and sharpness can be evaluated by the user or by the computer using an image analysis program.

the floater is in focus. In certain embodiments, the computer may analyze an image of the floater to determine if the floater is in focus. In other embodiments, the user may determine if the floater is in focus.

The SLO subsystem generates an SLO image of the floater at step 116. In certain embodiments, the SLO subsystem generates a plurality of images at different z-locations, and the computer generates a three-dimensional image from the plurality of images. The computer determines the xy-location of the floater at step 120. The xy-location may be determined by analyzing the SLO image of the floater in the vitreous.

The treatment laser subsystem directs a treatment laser beam towards the floater at step 122. For example, the treatment laser subsystem directs a laser pulse towards the floater. As another example, the treatment laser subsystem directs laser pulses that form a three-dimensional volume. The treatment laser subsystem substantially aligns the geometric center of the three-dimensional volume with the geometric center of the floater. The user may want to check for more floaters at step 124. If so, the method returns to step 110, where the scan region is placed at the retina to generate an SLO image of the retina to check if there are any more floater shadows, indicating the presence of floaters. If not, the method ends.

A component (such as control computer 26) of the systems and apparatuses disclosed herein may include an interface, logic, and/or memory, any of which may include computer hardware and/or software. An interface can receive input to the component and/or send output from the component, and is typically used to exchange information between, e.g., software, hardware, peripheral devices, users, and combinations of these. A user interface is a type of interface that a user can utilize to communicate with (e.g., send input to and/or receive output from) a computer. Examples of user interfaces include a display, Graphical User Interface (GUI), touchscreen, keyboard, mouse, gesture sensor, microphone, and speakers.

Logic can perform operations of the component. Logic may include one or more electronic devices that process data, e.g., execute instructions to generate output from input. Examples of such an electronic device include a computer, processor, microprocessor (e.g., a Central Processing Unit (CPU)), and computer chip. Logic may include computer software that encodes instructions capable of being executed by an electronic device to perform operations. Examples of computer software include a computer program, application, and operating system.

A memory can store information and may comprise tangible, computer-readable, and/or computer-executable storage medium. Examples of memory include computer memory (e.g., Random Access Memory (RAM) or Read Only Memory (ROM)), mass storage media (e.g., a hard disk), removable storage media (e.g., a Compact Disk (CD) or Digital Video or Versatile Disk (DVD)), database, network storage (e.g., a server), and/or other computer-readable media. Particular embodiments may be directed to memory encoded with computer software.

## Claims

1. An ophthalmic surgical laser system (10) for treating a floater in a vitreous of an eye, the eye having a retina, the system comprising:
a scanning laser ophthalmoscope, SLO, subsystem (20) comprising:
an SLO laser source (30) configured to provide an SLO laser beam with an SLO focal point; and
a pinhole filter conjugated to the SLO focal point;
a treatment laser subsystem (22) configured to provide a treatment laser beam with a treatment focal point, the treatment focal point spatially coinciding with the SLO focal point;
a scanner (40) configured to:
scan the SLO laser beam across a scan region; and
direct the treatment laser beam to an xy-location of the scan region;
a plurality of optical elements comprising a plurality of focusing lenses, the optical elements configured to:
aim the SLO laser beam and the treatment laser beam at substantially the same point of the scan region;
focus the SLO focal point to form the scan region at a z-scan location;
move the scan region in a z-direction to the z-scan location of the floater; and
focus the treatment focal point at the z-scan location of the floater; and
a computer (26) configured to:
receive an image of the floater from the SLO subsystem (20);
determine an xy-location of the floater; and
instruct the treatment laser subsystem (22) to direct the treatment laser beam towards the xy-location and the z-scan location of the floater.

2. The ophthalmic surgical laser system of Claim 1, the optical elements configured to move the scan region in the z-direction according to at least one of the following:
(i) the optical elements configured to move the scan region in the z-direction by changing the relative distance between the eye and at least one of the plurality of optical elements;
(ii) the optical elements configured to move the scan region in the z-direction by: changing the relative distance between the plurality of focusing lenses.

3. The ophthalmic surgical laser system of Claim 1, the optical elements:
further comprising a tunable lens configured to move the SLO focal point and the treatment focal point; and
configured to move the scan region in the z-direction by adjusting the tunable lens to move the SLO focal point and the treatment focal point in the z-direction.

4. The ophthalmic surgical laser system of Claim 1, the computer (26) configured to direct the SLO focal point to the xy-location of the floater by:
receiving user input indicating the xy-location of the floater.

5. The ophthalmic surgical laser system of Claim 1, the computer (26) configured to determine the xy-location of the floater by:
analyzing the image of the floater from the SLO subsystem (20); and
determining the xy-location of the floater from the image.

6. The ophthalmic surgical laser system of Claim 1:
the optical elements configured to move the scan region to the retina of the eye; and
the SLO subsystem (20) configured to generate an image of a shadow of the floater on the retina.

7. The ophthalmic surgical laser system of Claim 1, the treatment laser subsystem (22) configured to direct the treatment laser beam towards the xy-location and the z-scan location of the floater by:
directing a plurality of laser pulses at the floater.

8. The ophthalmic surgical laser system of Claim 1, the treatment laser subsystem (22) configured to direct the treatment laser beam towards the xy-location and the z-scan location of the floater by:
directing a plurality of laser pulses towards the floater, the plurality of pulses forming a three-dimensional volume, the three-dimensional volume covering 80% or more of a volume of the floater.

9. The ophthalmic surgical laser system of Claim 1, the computer (26) configured to:
analyze an image of a shadow on the retina of the eye from the SLO subsystem (20); and
determine whether the shadow is of a shadow of a significant floater.

10. The ophthalmic surgical laser system of Claim 1, the computer (26) configured to:
analyze an image of the floater from the SLO subsystem (20); and
determine whether the floater is in focus.

11. The ophthalmic surgical laser system of Claim 1, the computer (26) configured to perform at least one of the following:
track movement of the floater using tracking software; or
calculate a floater-to-retina distance according to changes in the system used to adjust the scan region between the retina and the floater.

12. The ophthalmic surgical laser system of Claim 11, the computer (26) configured to:
calculate the floater-to-retina distance according to at least one of the following:
(i) the computer (26) configured to: calculate the floater-to-retina distance according to a distance a plurality of system components move to adjust the scan region between the retina and the floater;
(ii) the computer (26) configured to:
calculate the floater-to-retina distance according to a difference in diopters used to adjust the scan region between the retina and the floater.

13. The ophthalmic surgical laser system of Claim 1, the computer (26) configured to:
calculate a retinal radiation exposure according to one or more of the following:
(i) the computer (26) configured to: calculate a retinal radiation exposure of the treatment laser beam focused at the z-scan location of the floater; and
perform an alarm response if the retinal radiation exposure exceeds a predetermined limit;
(ii) the computer (26) further configured to calculate the retinal radiation exposure EXP according to EXP= E/[(L*α)²π/4], where E represents a laser pulse energy, α represents a full convergence angle of the treatment laser beam focused at the z-scan location of the floater, and L represents a floater-to-retina distance calculated according to the z-scan location of the floater.

14. The ophthalmic surgical laser system of Claim 1:
the SLO subsystem (20) configured to generate a plurality of images of the eye at different z-scan locations; and
the computer (26) configured to generate a three-dimensional image from the plurality of images.

15. The ophthalmic surgical laser system of Claim 1, further comprising an ultrasonic sensor configured to monitor the position of the eye relative to an objective lens of the plurality of optical elements.

## Patentansprüche

1. Ophthalmisches chirurgisches Lasersystem (10) zum Behandeln eines Schwimmers in einem Glaskörper eines Auges, wobei das Auge eine Netzhaut aufweist, wobei das System Folgendes umfasst:
ein Subsystem (20) eines Scanlaser-Ophthalmoskops, SLO, das Folgendes umfasst:
eine SLO-Laserquelle (30), die dazu ausgelegt ist, einen SLO-Laserstrahl mit einem SLO-Brennpunkt bereitzustellen; und
ein Pinhole-Filter, das mit dem SLO-Brennpunkt konjugiert ist;
ein Behandlungslaser-Subsystem (22), das dazu ausgelegt ist, einen Behandlungslaserstrahl mit einem Behandlungsbrennpunkt bereitzustellen, wobei der Behandlungsbrennpunkt räumlich mit dem SLO-Brennpunkt zusammenfällt;
ein Scanner (40), der ausgelegt ist zum:
Scannen des SLO-Laserstrahls über einen Scanbereich; und Richten des Behandlungslaserstrahls auf eine xy-Position des Scanbereichs;
mehrere optische Elemente, die mehrere Fokussierlinsen umfassen, wobei die optischen Elemente ausgelegt sind zum:
Richten des SLO-Laserstrahls und des Behandlungslaserstrahls im Wesentlichen auf denselben Punkt des Scanbereichs;
Fokussieren des SLO-Brennpunkts, um den Scanbereich an einer z-Scanposition zu bilden;
Bewegen des Scanbereichs in eine z-Richtung zur z-Scanposition des Schwimmers; und
Fokussieren des Behandlungsbrennpunkts auf die z-Scanposition des Schwimmers; und
einen Computer (26), der ausgelegt ist zum:
Empfangen eines Bildes des Schwimmers von dem SLO-Subsystem (20);
Bestimmen einer xy-Position des Schwimmers; und
Anweisen des Bestrahlungslaser-Subsystems (22), den Behandlungslaserstrahl auf die xy-Position und die z-Scanposition des Schwimmers zu richten.

2. Ophthalmisches chirurgisches Lasersystem nach Anspruch 1, wobei die optischen Elemente dazu ausgelegt sind, den Scanbereich in die z-Richtung gemäß mindestens einem der Folgenden zu bewegen:
(i) den optischen Elementen, die dazu ausgelegt sind, den Scanbereich in die z-Richtung zu bewegen, indem der relative Abstand zwischen dem Auge und mindestens einem der mehreren optischen Elemente geändert wird;
(ii) den optischen Elementen, die dazu ausgelegt sind, den Scanbereich in die z-Richtung zu bewegen, durch: Ändern des relativen Abstands zwischen den mehreren Fokussierlinsen.

3. Ophthalmisches chirurgisches Lasersystem nach Anspruch 1, wobei die optischen Elemente:
ferner eine abstimmbare Linse umfassen, die dazu ausgelegt ist, den SLO-Brennpunkt und den Behandlungsbrennpunkt zu bewegen; und
dazu ausgelegt sind, den Scanbereich durch Anpassen der abstimmbaren Linse in die z-Richtung zu bewegen, um den SLO-Brennpunkt und den Behandlungsbrennpunkt in die z-Richtung zu bewegen.

4. Ophthalmisches chirurgisches Lasersystem nach Anspruch 1, wobei der Computer (26) dazu ausgelegt ist, den SLO-Brennpunkt auf die xy-Position des Schwimmers zu richten, durch:
Empfangen einer Benutzereingabe, die die xy-Position des Schwimmers angibt.

5. Ophthalmisches chirurgisches Lasersystem nach Anspruch 1, wobei der Computer (26) dazu ausgelegt ist, die xy-Position des Schwimmers zu bestimmen, durch:
Analysieren des Bildes des Schwimmers aus dem SLO-Subsystem (20); und
Bestimmen der xy-Position des Schwimmers aus dem Bild.

6. Ophthalmisches chirurgisches Lasersystem nach Anspruch 1:
wobei die optischen Elemente dazu ausgelegt sind, den Scanbereich zur Netzhaut des Auges zu bewegen; und
wobei das SLO-Subsystem (20) dazu ausgelegt ist, ein Bild eines Schattens des Schwimmers auf der Netzhaut zu erzeugen.

7. Ophthalmisches chirurgisches Lasersystem nach Anspruch 1, wobei das Behandlungslaser-Subsystem (22) dazu ausgelegt ist, den Behandlungslaserstrahl in Richtung der xy-Position und der z-Scanposition des Schwimmers zu richten durch:
Richten mehrerer Laserpulse auf den Schwimmer.

8. Ophthalmisches chirurgisches Lasersystem nach Anspruch 1, wobei das Behandlungslaser-Subsystem (22) dazu ausgelegt ist, den Behandlungslaserstrahl in Richtung der xy-Position und der z-Scanposition des Schwimmers zu richten durch:
Richten mehrerer Laserpulse auf den Schwimmer, wobei die mehreren Pulse ein dreidimensionales Volumen bilden, wobei das dreidimensionale Volumen 80 % oder mehr eines Volumens des Schwimmers bedeckt.

9. Ophthalmisches chirurgisches Lasersystem nach Anspruch 1, wobei der Computer (26) ausgelegt ist zum:
Analysieren eines Bildes eines Schattens auf der Netzhaut des Auges aus dem SLO-Subsystem (20); und
Bestimmen, ob der Schatten ein Schatten eines bedeutenden Schwimmers ist.

10. Ophthalmisches chirurgisches Lasersystem nach Anspruch 1, wobei der Computer (26) ausgelegt ist zum:
Analysieren eines Bildes des Schwimmers aus dem SLO-Subsystem (20); und
Bestimmen, ob der Schwimmer im Fokus ist.

11. Ophthalmisches chirurgisches Lasersystem nach Anspruch 1, wobei der Computer (26) dazu ausgelegt ist, mindestens eines der Folgenden durchzuführen:
Verfolgen der Bewegung des Schwimmers mit Hilfe der Tracking-Software; oder
Berechnen eines Abstands zwischen dem Schwimmer und der Netzhaut gemäß den Änderungen im System, das zum Anpassen des Scanbereichs zwischen der Netzhaut und dem Schwimmer verwendet wird.

12. Ophthalmisches chirurgisches Lasersystem nach Anspruch 11, wobei der Computer (26) ausgelegt ist zum:
Berechnen des Abstands zwischen Schwimmer und Netzhaut gemäß mindestens einem der Folgenden:
(i) dem Computer (26), der ausgelegt ist zum: Berechnen des Abstands zwischen Schwimmer und Netzhaut gemäß einem Abstand, über den sich mehrere Systemkomponenten bewegen, um den Scanbereich zwischen der Netzhaut und dem Schwimmer anzupassen;
(ii) dem Computer (26), der ausgelegt ist zum:
Berechnen des Abstands zwischen Schwimmer und Netzhaut gemäß einer Differenz in Dioptrien, die zum Anpassen des Scanbereichs zwischen der Netzhaut und dem Schwimmer verwendet werden.

13. Ophthalmisches chirurgisches Lasersystem nach Anspruch 1, wobei der Computer (26) ausgelegt ist zum:
Berechnen einer retinalen Strahlenexposition gemäß einem oder mehreren der Folgenden:
(i) dem Computer (26), der ausgelegt ist zum: Berechnen einer retinalen Strahlenexposition des Behandlungslaserstrahls, der an der z-Scanposition des Schwimmers fokussiert ist; und
Durchführen einer Alarmreaktion, wenn die retinale Strahlenexposition eine vorbestimmte Grenze überschreitet;
(ii) dem Computer (26), der ferner dazu ausgelegt ist, die retinale Strahlenexposition EXP gemäß EXP= E/[(L*α)²π/4] zu berechnen, wobei E eine Laserpulsenergie repräsentiert, α einen vollen Konvergenzwinkel des Behandlungslaserstrahls repräsentiert, der an der z-Scanposition des Schwimmers fokussiert ist, und L einen Abstand zwischen Schwimmer und Netzhaut repräsentiert, der gemäß der z-Scanposition des Schwimmers berechnet wird.

14. Ophthalmisches chirurgisches Lasersystem nach Anspruch 1:
wobei das SLO-Subsystem (20) dazu ausgelegt ist, mehrere Bilder des Auges an verschiedenen z-Scanpositionen zu erzeugen; und
wobei der Computer (26) dazu ausgelegt ist, ein dreidimensionales Bild aus den mehreren Bildern zu erzeugen.

15. Ophthalmisches chirurgisches Lasersystem nach Anspruch 1, das ferner einen Ultraschallsensor umfasst, der dazu ausgelegt ist, die Position des Auges relativ zu einer Objektivlinse der mehreren optischen Elemente zu überwachen.

## Revendications

1. Système laser chirurgical ophtalmique (10) pour traiter un flotteur dans une humeur vitrée d'un œil, l'œil ayant une rétine, le système comprenant :
un sous-système d'ophtalmoscope à laser de balayage, SLO (20) comprenant :
une source laser SLO (30) configurée pour fournir un faisceau laser SLO avec un point focal SLO ; et
un filtre sténopé conjugué au point focal SLO ;
un sous-système laser de traitement (22) configuré pour fournir un faisceau laser de traitement avec un point focal de traitement, le point focal de traitement coïncidant spatialement avec le point focal SLO ;
un dispositif de balayage (40) configuré pour :
balayer le faisceau laser SLO sur une région de balayage ; et
diriger le faisceau laser de traitement vers un emplacement xy de la région de balayage ;
une pluralité d'éléments optiques comprenant une pluralité de lentilles de focalisation, les éléments optiques étant configurés pour :
viser le faisceau laser SLO et le faisceau laser de traitement sensiblement au même point de la région de balayage ;
focaliser le point focal SLO pour former la région de balayage à un emplacement de balayage z ;
déplacer la région de balayage dans la direction z jusqu'à l'emplacement de balayage z du flotteur ; et
focaliser le point focal de traitement sur l'emplacement de balayage z du flotteur ; et
un ordinateur (26) configuré pour :
recevoir une image du flotteur du sous-système SLO (20) ; déterminer un emplacement xy du flotteur ; et
ordonner au sous-système laser de traitement (22) de diriger le faisceau laser de traitement vers l'emplacement xy et l'emplacement de balayage z du flotteur.

2. Système laser chirurgical ophtalmique selon la revendication 1, les éléments optiques étant configurés pour déplacer la région de balayage dans la direction z selon au moins l'une des situations suivantes :
(i) les éléments optiques sont configurés pour déplacer la région de balayage dans la direction z en modifiant la distance relative entre l'œil et au moins un parmi la pluralité d'éléments optiques ;
(ii) les éléments optiques sont configurés pour déplacer la région de balayage dans la direction z par : la modification de la distance relative entre la pluralité de lentilles de focalisation.

3. Système laser chirurgical ophtalmique selon la revendication 1, les éléments optiques :
comprenant en outre une lentille accordable configurée pour déplacer le point focal SLO et le point focal de traitement ; et
configurée pour déplacer la région de balayage dans la direction z en réglant la lentille accordable pour déplacer le point focal SLO et le point focal de traitement dans la direction z.

4. Système laser chirurgical ophtalmique selon la revendication 1, l'ordinateur (26) étant configuré pour diriger le point focal SLO vers l'emplacement xy du flotteur par :
la réception d'une entrée utilisateur indiquant l'emplacement xy du flotteur.

5. Système laser chirurgical ophtalmique selon la revendication 1, l'ordinateur (26) étant configuré pour déterminer l'emplacement xy du flotteur par :
l'analyse de l'image du flotteur à partir du sous-système SLO (20) ; et
la détermination de l'emplacement xy du flotteur à partir de l'image.

6. Système laser chirurgical ophtalmique selon la revendication 1 :
les éléments optiques étant configurés pour déplacer la région de balayage vers la rétine de l'œil ; et
le sous-système SLO (20) étant configuré pour générer une image d'une ombre du flotteur sur la rétine.

7. Système laser chirurgical ophtalmique selon la revendication 1, le sous-système laser de traitement (22) étant configuré pour diriger le faisceau laser de traitement vers l'emplacement xy et l'emplacement de balayage z du flotteur par :
l'orientation d'une pluralité d'impulsions laser sur le flotteur.

8. Système laser chirurgical ophtalmique selon la revendication 1, le sous-système laser de traitement (22) étant configuré pour diriger le faisceau laser de traitement vers l'emplacement xy et l'emplacement de balayage z du flotteur par :
l'orientation d'une pluralité d'impulsions laser vers le flotteur, la pluralité d'impulsions formant un volume tridimensionnel, le volume tridimensionnel couvrant 80 % ou plus d'un volume du flotteur.

9. Système laser chirurgical ophtalmique selon la revendication 1, l'ordinateur (26) étant configuré pour :
analyser une image d'une ombre sur la rétine de l'œil à partir du sous-système SLO (20) ; et
déterminer si l'ombre est d'une ombre d'un flotteur important.

10. Système laser chirurgical ophtalmique selon la revendication 1, l'ordinateur (26) étant configuré pour :
analyser une image du flotteur provenant du sous-système SLO (20) ; et
déterminer si le flotteur est au point.

11. Système laser chirurgical ophtalmique selon la revendication 1, l'ordinateur (26) étant configuré pour réaliser au moins une des actions suivantes :
suivre le mouvement du flotteur à l'aide du logiciel de suivi ; ou
calculer une distance entre le flotteur et la rétine en fonction des changements du système utilisé pour ajuster la région de balayage entre la rétine et le flotteur.

12. Système laser chirurgical ophtalmique selon la revendication 11, l'ordinateur (26) étant configuré pour :
calculer la distance entre le flotteur et la rétine selon au moins l'une des situations suivantes :
(i) l'ordinateur (26) est configuré pour : calculer la distance entre le flotteur et la rétine en fonction d'une distance de déplacement d'une pluralité de composants du système pour ajuster la région de balayage entre la rétine et le flotteur ;
(ii) l'ordinateur (26) est configuré pour :
calculer la distance entre le flotteur et la rétine en fonction d'une différence de dioptries utilisée pour ajuster la région de balayage entre la rétine et le flotteur.

13. Système laser chirurgical ophtalmique selon la revendication 1, l'ordinateur (26) étant configuré pour :
calculer une exposition au rayonnement rétinien selon une ou plusieurs des situations suivantes :
(i) l'ordinateur (26) est configuré pour : calculer une exposition au rayonnement rétinien du faisceau laser de traitement focalisé à l'emplacement de balayage z du flotteur ; et
effectuer une réponse d'alarme si l'exposition au rayonnement rétinien dépasse une limite prédéterminée ;
(ii) l'ordinateur (26) est en outre configuré pour calculer l'exposition au rayonnement rétinien EXP selon EXP= E/[(L*α)²π/4], E représentant une énergie d'impulsion laser, α représentant un angle de convergence totale du faisceau laser de traitement focalisé à l'emplacement de balayage z du flotteur, et L représentant une distance entre le flotteur et la rétine calculée en fonction de l'emplacement de balayage z du flotteur.

14. Système laser chirurgical ophtalmique selon la revendication 1 :
le sous-système SLO (20) étant configuré pour générer une pluralité d'images de l'œil à différents emplacements de balayage z ; et
l'ordinateur (26) étant configuré pour générer une image tridimensionnelle à partir de la pluralité d'images.

15. Système laser chirurgical ophtalmique selon la revendication 1, comprenant en outre un capteur ultrasonore configuré pour surveiller la position de l'œil par rapport à une lentille d'objectif de la pluralité d'éléments optiques.
